# EUROPEAN PATENT APPLICATION

(11) **EP 2 724 740 A1**
(43) Date of publication of application: **30.04.2014**
(21) Application number: 12802256.3
(22) Date of filing: 11.06.2012
(51) Int. Cl.: A61M 5/28, A61M 5/315

(54) **PRE-FILLED SYRINGE**

(30) Priority: 21.06.2011 JP 2011137621
(71) Applicant: Suzuken Co., Ltd., Aichi 461-8701 (JP); Taisei Kako Co., Ltd., Kita-ku Osaka-shi Osaka 531-0072 (JP)
(72) Inventor: SUZUKI, Ichiro, Nagoya-shi Aichi 461-8701 (JP); OGAWA, Yukihiro, Ibaraki-shi Osaka 567-0054 (JP)
(74) Representative: TBK
(86) International application number: PCT/JP2012/064905
(87) International publication number: WO 2012/176643

(57) **Abstract**

Provided is a very safe pre-filled syringe that reduces the time and efforts required in administering the injection. In the pre-filled syringe, a needle unit (1) attached to a vial includes: a substantially columnar retaining member (10) with needles at both ends; a holder member (11) holding the retaining member (10) with three pillar-shaped parts (111); and a holder member (12) holding the retaining member (10) with three pillar-shaped parts (121) and having an attachment portion (125) for the vial. The needle unit (1) is axially retractable when the pillar-shaped parts (111, 121) are alternately arranged in the circumferential direction, and is not axially retractable when distal end surfaces of the pillar-shaped parts (111) in one holder member face distal end surfaces of the pillar-shaped parts (121) in the other holder member. Axially retracting the needle unit (1) makes an injection needle (100A) project, and makes a perforation needle (100B) project inside the vial.

## Description

### Technical Field

The present invention relates to a disposable pre-filled syringe filled with a drug solution.

### Background Art

Pre-filled syringes, which have been filled in advance with a drug solution used for medical treatment and the like, are conventionally utilized in medical facilities such as hospitals. When a general syringe is used to inject a drug solution contained in an ampule and a vial, it is required to aspirate the drug solution into the syringe prior to administration of the injection. On the other hand, when a pre-filled syringe is used, this aspiration need not be performed, and therefore the workload associated with administration of the injection can be alleviated. The use of pre-filled syringes is effective in obviating medical accidents, such as incorrect administration caused by mix-ups of drug solutions, and overdose caused by using an inaccurate amount of solution. The use of pre-filled syringes can also reduce the risks of administering the injection, such as contamination by foreign substances and bacteria when transferring drug solutions from ampules and the like to syringes.

Examples of pre-filled syringes include a pre-filled syringe that is configured similarly to a general syringe (see, for example, PTL 1), and a pre-filled syringe that is configured such that a needle unit is attached to a container filled with a drug solution (see, for example, PTL 2). The former pre-filled syringe includes: a barrel that has a cylindrical shape and is already filled with a drug solution; a gasket that closes an open end side of the barrel in a manner movable in the forward direction; and an injection needle connection unit at a distal end side of the barrel closed by a cap. In the latter pre-filled syringe, a gasket closes an opening of the drug solution container in a manner movable in the forward direction, and the needle unit is attached to the gasket in such a manner that it can be pushed into the drug solution container.

With the former pre-filled syringe, the injection can be administered through a procedure similar to a procedure for the general injector by removing the cap at the distal end of the barrel and by attaching an injection needle. With the latter pre-filled syringe, the drug solution can be placed in an injectable state by attaching the needle unit to the drug solution container, and the drug solution can be injected by moving the gasket in the forward direction through the act of pushing the needle unit.

However, the aforementioned conventional pre-filled syringes have the following problems. Administration of the injection requires, for example, the work of attaching the injection needle and attaching the needle unit to the drug solution container. This gives rise to the problems that the time and efforts required in administering the injection are not sufficiently alleviated and safety is not sufficiently ensured.

### Citation List

### Patent Literature

Patent Literature 1: JP 2008-307237A
Patent Literature 2: JP 2002-172166A

### Summary of Invention

### Problems to be Solved by the Invention

The present invention has been made in view of the above conventional problems, and aims to provide a very safe pre-filled syringe that alleviates the time and efforts required in administering the injection.

### Means for Solving the Problems

The present invention provides a pre-filled syringe including: a drug solution container that is filled with a drug solution and has a shape of a cylinder with a bottom; a closing member that closes an opening of the drug solution container in a manner movable in an axial direction, which is equivalent to a boring direction of the cylindrical shaped drug solution container; and a needle housing unit that is attached to the closing member so as to allow the closing member to be pushed into the drug solution container. The needle housing unit includes: a substantially columnar retaining member with an injection needle projecting from one end thereof in the axial direction and a perforation needle projecting from the other end thereof in the axial direction; a first holder member provided with a first hollow portion having a bottom and with a first slider portion, the first slider portion being able to hold the retaining member along the axial direction, which is equivalent to a direction from a bottom side toward an opening side of the first hollow portion, using at least two pillar-shaped parts, the at least two pillar-shaped parts extending from the bottom side of the first hollow portion along the axial direction and circumscribing an outer circumferential surface of the retaining member; and a second holder member provided with a second hollow portion having a bottom, with a second slider portion, and with an attachment portion, the second slider portion being able to hold the retaining member using at least two pillar-shaped parts that extend from a bottom side of the second hollow portion along the axial direction, which is equivalent to a direction from a bottom side toward an opening side of the second hollow portion, and that circumscribe the outer circumferential surface of the retaining member, the attachment portion being provided on a bottom end of the second holder member for the closing member. The first holder member and the second holder member are rotatable relative to each other around the retaining member in a state where each pillar-shaped part in the first slider portion or the second slider portion holding the retaining member does not overlap with any of the pillar-shaped parts in the other slider portion in the axial direction. In a state where the pillar-shaped parts in the slider portions are arranged alternately around the retaining member, the first holder member and the second holder member are retractable in the axial direction through insertion of one of the holder members into the hollow portion in the other holder member, whereas in a state where distal end surfaces of the pillar-shaped parts in one of the slider portions face distal end surfaces of the pillar-shaped parts in the other slider portion, the first holder member and the second holder member are not retractable in the axial direction. When the first holder member and the second holder member holding the retaining member are retracted in the axial direction, the perforation needle penetrates through the closing member and projects inside the drug solution container, and the injection needle projects toward the outside.

In the pre-filled syringe according to the present invention, the needle housing unit can shift between an axially retractable state and an axially non-retractable state in accordance with the positions of the first and second holder members associated with the relative rotation. In the axially non-retractable state, distal end surfaces of the pillar-shaped parts in one holder member face distal end surfaces of the pillar-shaped parts in the other holder member, and the retraction in the axial direction is restricted with high reliability. In this state, the injection needle does not project toward the outside inadvertently, and therefore the occurrence of needlestick accidents can be prevented with high reliability.

When administering the injection, the needle housing unit is retracted in the axial direction by pushing one holder member into the other holder member. As a result, the perforation needle projects from the rear end of the needle housing unit in vicinity to the drug solution container, and the injection needle projects to the outside from the distal end of the needle housing unit. The perforation needle penetrates through the closing member and reaches the inside of the drug solution container. By pushing the entire needle housing unit into the drug solution container in this state, the closing member moves in the forward direction inside the drug solution container. This forward movement allows the drug solution to be injected from the injection needle.

When disposing the pre-filled syringe after administering the injection, for example, the injection needle that was used for the administration can be housed by extending the first and second holder members in the axial direction. Furthermore, the needle housing unit can be placed again in the axially non-retractable state by rotating the first and second holder members relative to each other such that the distal end surfaces of the pillar-shaped parts in one holder member face the distal end surfaces of the pillar-shaped parts in the other holder member. When the needle housing unit is placed again in this state, the possibility of inadvertent projection of the injection needle can be suppressed with high reliability during the subsequent handling.

As described above, the pre-filled syringe according to the present invention is a very safe and excellent product that reduces the time and efforts required in administering the injection.

In the pre-filled syringe according to the present invention, the number of the pillar-shaped parts in each of the first and second slider portions can be set to any number, such as two, three, and four.

In one preferred aspect of the present invention, the pre-filled syringe includes a surrounding sleeve having a substantially cylindrical shape, the surrounding sleeve restricting a relative rotation of the first holder member and the second holder member around the retaining member in a state where the surrounding sleeve surrounds the first slider portion and the second slider portion.

For example, restricting the relative rotation of the first and second holder members can suppress the possibility that the injection needle cartridge shifts inadvertently from the axially non-retractable state to the axially retractable state. By preventing such an inadvertent shift to the retractable state, needlestick accidents and the like can be prevented with high reliability.

In another preferred aspect of the present invention, the pre-filled syringe is configured as follows: the needle housing unit is in an axially non-retractable state in a manufactured state, and is placed in an axially retractable state by rotating the first holder member and the second holder member relative to each other around the retaining member during use, the axially non-retractable state being a state where the needle housing unit is not retractable in the axial direction, and the axially retractable state being a state where the needle housing unit is retractable in the axial direction; after use, the needle housing unit can be placed in the axially non-retractable state again by rotating the first holder member and the second holder member relative to each other after extending the first holder member and the second holder member in the axial direction to positions where each pillar-shaped part in the first slider portion or the second slider portion does not overlap with any of the pillar-shaped parts in the other slider portion in the axial direction; and the surrounding sleeve allows the relative rotation of the first holder member and the second holder member around the retaining member in the manufactured state, and restricts the relative rotation of the first holder member and the second holder member once the needle housing unit has shifted from the axially retractable state to the axially non-retractable state again after use.

In this case, once the needle housing unit has been placed in the axially non-retractable state after administering the injection, the relative rotation of the first and second holder members can be restricted. This restriction of the relative rotation makes it possible to reliably prevent the possibility that the first and second holder members are placed back in the axially retractable state. In this way, after the injection, needlestick accidents and the like of the pre-filled syringe can be obviated, and the safety of the pre-filled syringe can be ensured to a great extent.

In yet another preferred aspect of the present invention, the pre-filled syringe is configured such that the surrounding sleeve restricts a maximum extended length of the first holder member and the second holder member in the axial direction.

Surrounding the first and second slider portions with this surrounding sleeve reduces the possibility that the first and second holder members are extended so long in the axial direction that one holder member falls from the other holder member. This can obviate the possibility that the retaining member provided with the injection needle and the like is exposed to the outside.

In yet another preferred aspect of the present invention, the pre-filled syringe is configured as follows: the first holder member allows the second holder member to be inserted into the first hollow portion, and has finger grips on an outer circumferential side thereof, the finger grips allowing a user to place fingers therearound when administering an injection; and the injection can be administered with fingers placed on a bottom surface of the drug solution container and around the finger grips.

The finger grips may be arranged in various ways. For example, the finger grips may be arranged in opposition to each other at two places on the outer circumferential surface of the first holder member. The injection can be administered with a procedure that is substantially similar to a procedure for general injectors by, for example, placing a pointing finger and a middle finger around the finger grips and placing a thumb on the bottom surface of the drug solution container.

### Brief Description of Drawings

Fig. 1 is a perspective view showing a pre-filled syringe in a manufactured state according to a working example.
Fig. 2 is a perspective view showing the pre-filled syringe in an injection state according to the working example.
Fig. 3 is a cross-sectional view showing a cross-sectional configuration of a vial closed by a gasket according to the working example.
Fig. 4 is a perspective view showing an assembly configuration of a needle unit in the manufactured state according to the working example.
Fig. 5 is a perspective view showing the needle unit in the manufactured state according to the working example.
Fig. 6 is a perspective view showing the assembly configuration of the needle unit in the manufactured state according to the working example.
Fig. 7 shows a surrounding sleeve according to the working example.
Fig. 8 is a cross-sectional view showing a cross-sectional configuration of the surrounding sleeve according to the working example.
Fig. 9 is explanatory views showing operations of the needle unit according to the working example.
Fig. 10 is a perspective view showing the assembly configuration of the needle unit in the injection state according to the working example.
Fig. 11 is a perspective view showing the needle unit in the axially retractable state according to the working example.
Fig. 12 is a perspective view showing the needle unit in the injection state according to the working example.
Fig. 13 is a cross-sectional view showing a first pre-filled syringe in a manufactured state according to one reference example.
Fig. 14 is a cross-sectional view showing the first pre-filled syringe in an injection state according to the reference example.
Fig. 15 is a cross-sectional view showing a second pre-filled syringe in the state where preparation for the injection has completed according to another reference example.
Fig. 16 is a cross-sectional view showing the second pre-filled syringe in an injection state according to the other reference example.

### Description of Embodiment

An embodiment of the present invention will now be described in detail using the following working example.

### (Working Example)

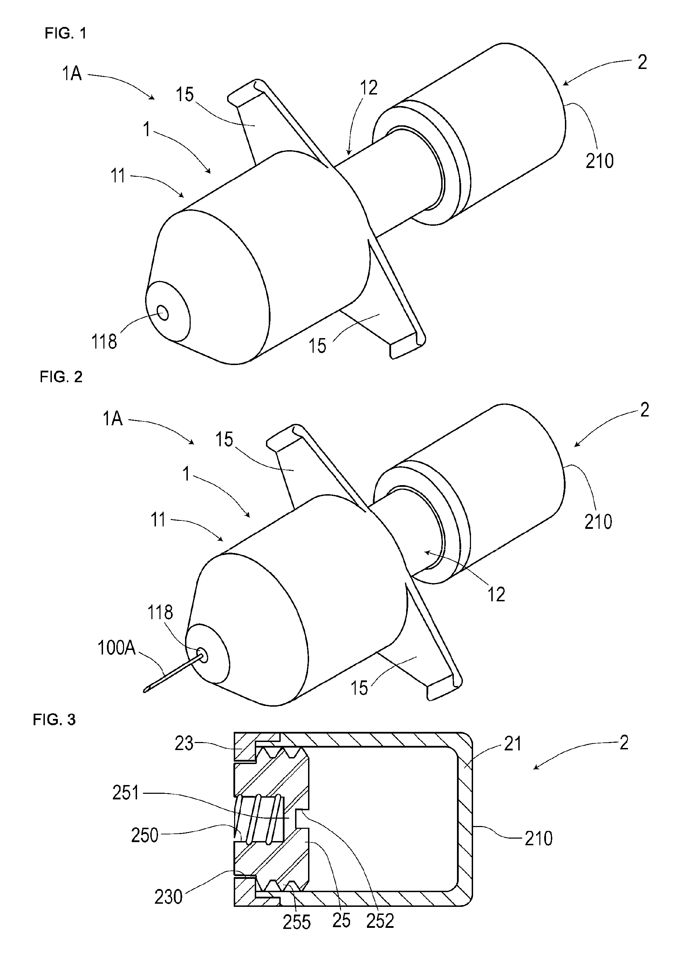

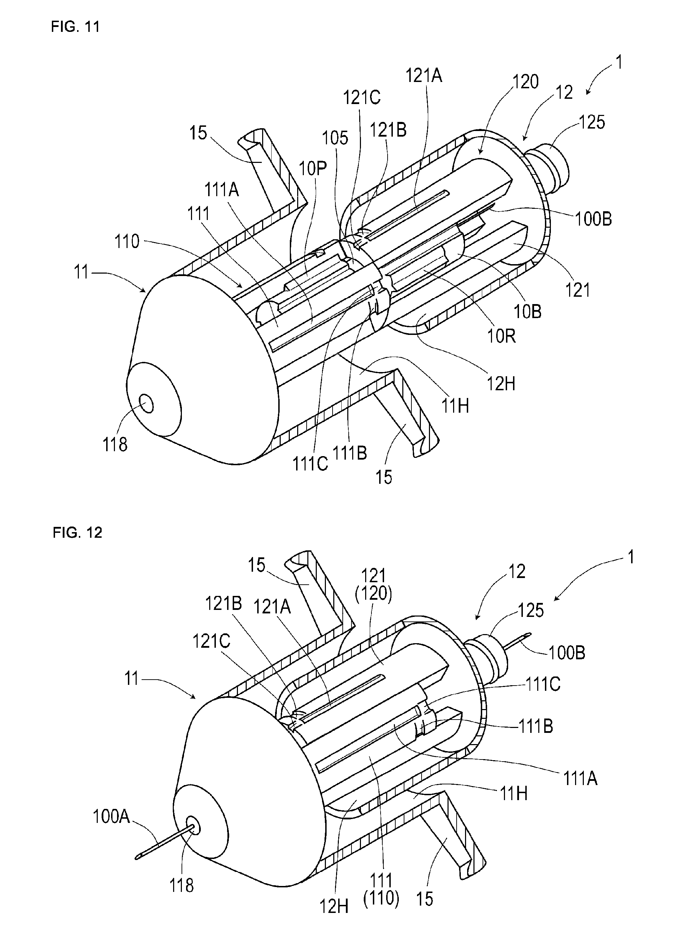

The present example relates to a disposable pre-filled syringe 1A (an injector that is already filled with a drug solution). Specifics of this pre-filled syringe 1A will be described below with reference to Figs. 1 to 12.

As shown in Figs. 1 to 3, the pre-filled syringe 1A according to the present example has a vial 2 (drug solution container), a gasket 25 (closing member), and a needle unit 1 (needle housing unit). The vial 2 has a shape of a cylinder with a bottom and is filled with the drug solution. The gasket 25 closes an opening in a manner movable in the boring direction of the cylindrical shaped vial 2 so as to push the drug solution out. The needle unit 1 is attached to the gasket 25 in such a manner that the gasket 25 can be pushed into the vial 2. The pre-filled syringe 1A in a manufactured state has a total length of approximately 60 mm, and a maximum diameter of approximately 15 mm excluding finger grips 15.

The pre-filled syringe 1A in the manufactured state is covered in its entirety by a film made of polypropylene (not shown in the drawings), and is thus kept in a sterile condition. It should be noted that the external film is not limited to being made of polypropylene, and may be made of various materials such as polyethylene, polyvinylidene chloride, and polyethylene terephthalate.

As shown in Figs. 1 to 3, the vial 2 is a container having a shape of a cylinder with a bottom, and is to be filled with the drug solution. The vial 2 is closed by the gasket 25 inserted therein. A fall prevention portion 23 for preventing the gasket 25 from falling is attached to an open end of the vial 2. This fall prevention portion 23 has an opening 230 with a diameter smaller than the inner diameter of a body 21 of the vial 2.

As shown in Fig. 3, the gasket 25 is a substantially columnar member that is inserted into the vial 2 (body 21) and is made of butyl rubber or elastomer. Ribs 255 are provided on the outer circumferential surface of the gasket 25 in three places along the axial direction (the boring direction of the cylindrical shaped vial 2). Each rib 255 projects in the form of a convexity extending along the entire circumference. This gasket 25 functions as a piston that moves in the forward direction toward the bottom side of the vial 2.

An attachment hole 250 with a bottom is provided on an end surface of the gasket 25 that is exposed to the outside when the gasket 25 is inserted into the vial 2. A thread is provided on the internal circumferential surface of the attachment hole 250. The needle unit 1 is threaded into and attached to the attachment hole 250. A small-diameter hole 252 with a bottom is provided at the center of the other end surface of the gasket 25. This small-diameter hole 252 is bored so as to face the attachment hole 250 with a thin wall 251 therebetween.

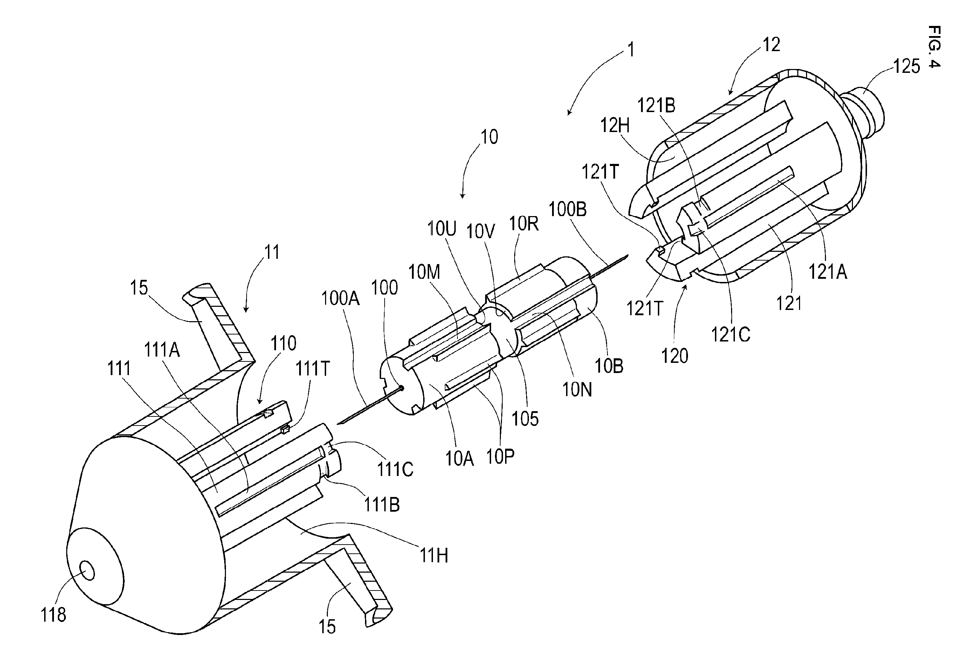

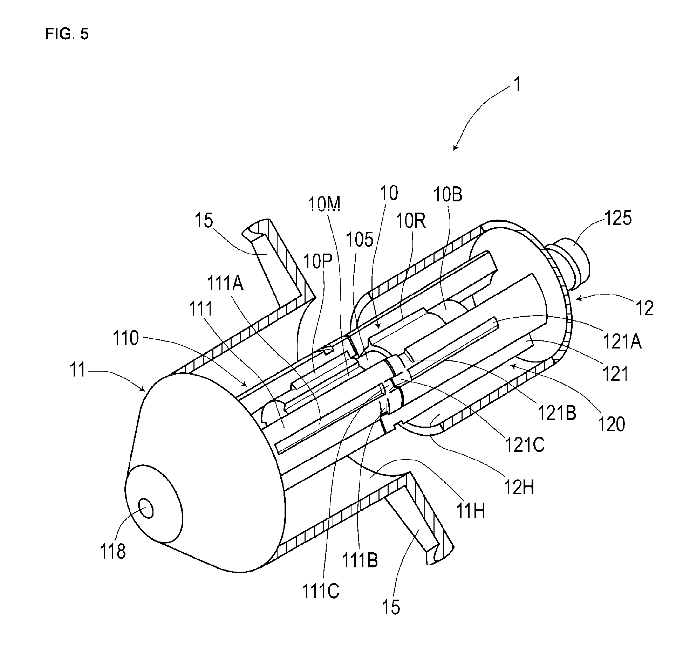

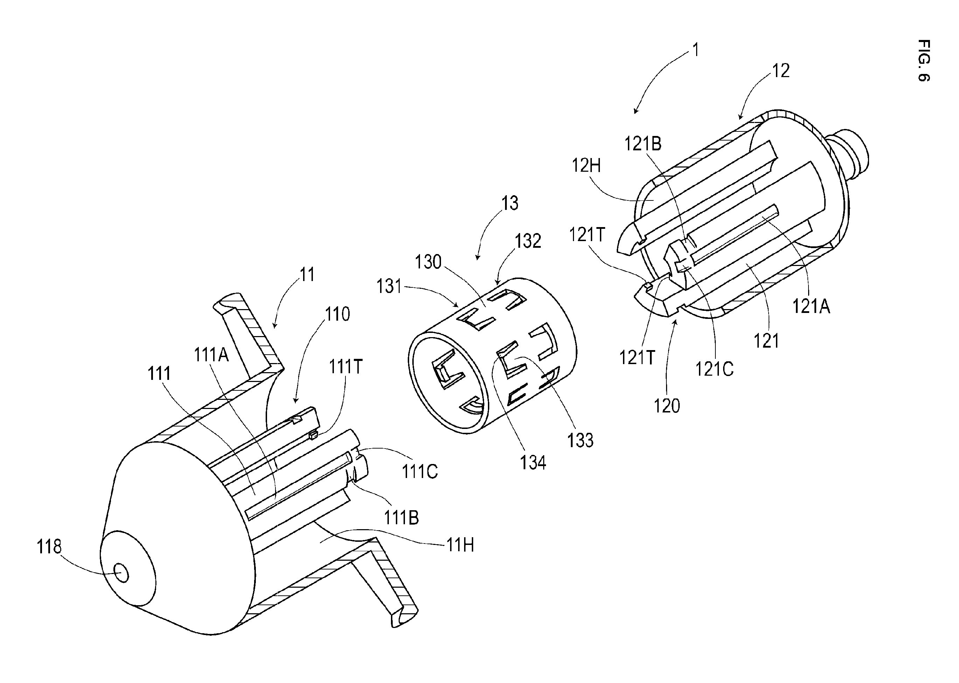

As shown in Figs. 1, 2, and 4 to 6, the needle unit 1 is composed of a substantially columnar retaining member 10, a first holder member 11, a second holder member 12, and a surrounding sleeve 13. An injection needle 100A and a perforation needle 100B project from both ends of the retaining member 10. The first holder member 11 and the second holder member 12 house the retaining member 10 therein. The surrounding sleeve 13 is housed in the first holder member 11 and the second holder member 12 while surrounding the outer circumferential side of the retaining member 10. It should be noted that the surrounding sleeve 13 is omitted from Figs. 4 and 5, and the retaining member 10 is omitted from Fig. 6.

In the needle unit 1, the first holder member 11 and the second holder member 12 are coaxially joined via the retaining member 10. The needle unit 1 is retractable in the axial direction due to a configuration in which the second holder member 12 is inserted and housed in the first holder member 11 at the distal end side. Axially retracting the needle unit 1 with the vial 2 attached thereto allows the perforation needle 100B to project inside the vial 2 via the gasket 25, and allows the injection needle 100A to project toward the outside (see Fig. 2). By pushing the needle unit 1 into the vial 2 in this state, the gasket 25 moves in the forward direction, and therefore the drug solution can be injected from the injection needle 100A.

As shown in Fig. 4, the retaining member 10 is a substantially columnar member made of polycarbonate. A stainless steel tube penetrates through the retaining member 10 along the central axis of the retaining member 10. This stainless steel tube projects from both axial ends of the retaining member 10 and constitutes the injection needle 100A and the perforation needle 100B. The injection needle 100A on one side pierces through a site of injection, such as a human skin. The perforation needle 100B on the other side penetrates through the wall 251 in the gasket 25 (Fig. 3).

It should be noted that the injection needle 100A and the perforation needle 100B are not limited to being made of stainless steel, and may be made of a resin material. If the injection needle 100A and the like are made of resin, post-use disposal is further facilitated. If the injection needle 100A and the like are made of biodegradable plastic, disposal is facilitated as well.

As shown in Fig. 4, the retaining member 10 according to the present example has a small-diameter portion 105, which is arranged at an axially intermediate portion thereof, between end surfaces 10U and 10V. A first shaft portion 10A and a second shaft portion 10B, which are substantially equal in diameter, are formed on both axial sides of the small-diameter portion 105. This retaining member 10 is assembled such that the first shaft portion 10A is located on the side of the first holder member 11, and the second shaft portion 10B is located on the side of the second holder member 12.

As shown in Fig. 4, ridge portions 10P projecting toward the outer circumferential side are provided on the outer circumferential surface of the first shaft portion 10A at six places that are located at a substantially equal interval in the circumferential direction. Each ridge portion 10P extends along the axial direction. The outer circumferential surface of the first shaft portion 10A has six surface regions that are each arranged between the ridge portions 10P neighboring in the circumferential direction. Advance/retreat grooves 10M extending along the axial direction are provided in accordance with the same specifications on three of the six surface regions, more specifically, alternating surface regions out of the six surface regions.

As shown in Fig. 4, ridge portions 10R projecting toward the outer circumferential side are provided on the outer circumferential surface of the second shaft portion 10B at three places that are located at a substantially equal interval in the circumferential direction. Each ridge portion 10R extends along the axial direction. The outer circumferential surface of the second shaft portion 10B has three surface regions that are each arranged between the ridge portions 10R neighboring in the circumferential direction. Each of these three surface regions spans across approximately 120 degrees in the circumferential direction. Furthermore, advance/retreat grooves 10N extending along the axial direction are provided on the outer circumferential surface of the second shaft portion 10B at three places that are located at a substantially equal interval in the circumferential direction. Each advance/retreat groove 10N in the second shaft portion 10B is offset with respect to the corresponding advance/retreat grooves 10M in the first shaft portion 10A by approximately 60 degrees.

As shown in Figs. 4 to 6, the first holder member 11, which is made of polycarbonate, has a shape of a substantial cylinder with a bottom. The pair of finger grips 15 is provided at the open end of the first holder member 11. The finger grips 15 are arranged at opposite positions so as to project toward the outer circumferential side. This holder member 11 is assembled in such a manner that the bottom side thereof is located at the distal end side of the pre-filled syringe 1A. A bottom end portion of the holder member 11 has a tapered shape with a narrow end in external appearance. A projection hole 118 from which the injection needle 100A projects is bored on the distal end surface of this bottom end portion of the holder member 11. A first hollow portion 11H, which is the internal space of this holder member 11, has an inner diameter substantially equal to an outer diameter of the vial 2 so as to allow the vial 2 to be inserted into the first hollow portion 11H.

A first slider portion 110 that can hold the retaining member 10 is provided in the first hollow portion 11H. This slider portion 110 is composed of three pillar-shaped parts 111 that extend from the bottom side of the hollow portion 11H along the axial direction (a direction toward the open end). The three pillar-shaped parts 111 are all formed in accordance with the same specifications, and provided at three positions at an equal interval in the circumferential direction with the axis of the holder member 11 serving as the center. The three pillar-shaped parts 111 form an inner circumferential space with a substantially circular cross-section. The slider portion 110 holds the retaining member 10 in this inner circumferential space with the pillar-shaped parts 111 circumscribing the outer circumferential surface of the retaining member 10.

As shown in Figs. 3 to 6, the second holder member 12, which is made of polycarbonate, has a shape of a substantial cylinder with a bottom. An attachment portion 125 with a smaller diameter stands on an end surface of the second holder member 12 at the bottom side thereof. This holder member 12 is formed to be smaller in diameter than the opening 230 of the vial 2. The attachment portion 125 has a thread on the outer circumferential surface thereof so that it can be threaded into the attachment hole 250 of the gasket 25. The attachment portion 125 is threaded into the attachment hole 250 until the end surface of the holder member 12 on which the attachment portion 125 stands is in close contact with the gasket 25.

The inner diameter of a second hollow portion 12H, which is the internal space of the second holder member 12, is set so as to allow the surrounding sleeve 13 to be inserted into the second hollow portion 12H. A second slider portion 120 that can hold the retaining member 10 is provided in the second hollow portion 12H. This slider portion 120 is composed of three pillar-shaped parts 121 that extend from the bottom side of the hollow portion 12H along the axial direction (a direction toward the open end). The three pillar-shaped parts 121 are all formed in accordance with the same specifications as the above-described pillar-shaped parts 111 of the first holder member 11, and provided at three positions at an equal interval in the circumferential direction similarly to the pillar-shaped parts 111. The three pillar-shaped parts 121 form an inner circumferential space with a substantially circular cross-section. The slider portion 120 holds the retaining member 10 in this inner circumferential space with the pillar-shaped parts 121 circumscribing the outer circumferential surface of the retaining member 10.

When the pillar-shaped parts 111, 121 are arranged alternately in the circumferential direction, they fill the gaps therebetween without overlapping with one another, and therefore form a substantially complete circular ring in cross-section. In the state where the pillar-shaped parts 111, 121 are arranged alternately in the circumferential direction, the holder members 11, 12 are retractable in the axial direction with the pillar-shaped parts 111, 121 engaging with one another in a comb teeth form.

As shown in Figs. 4 to 6, the pillar-shaped parts 111, 121 have three types of grooves 111A to 111C, 121A to 121C on the outer circumferential surfaces thereof, and projections 111T, 121T on the inner circumferential surfaces of the distal ends thereof.
Each projection 111T, 121T, which projects toward the inner circumferential side, is formed in one place on the inner circumferential surface of the corresponding pillar-shaped parts 111, 121. The inner diameter formed by projecting surfaces of the three projections 111T, 121T of the holder member 11, 12 is substantially the same as the outer diameter of the small-diameter portion 105 of the retaining member 10. When the holder member 11 advances/retreats in the axial direction with respect to the retaining member 10, the projections 111T of the first holder member 11 advance/retreat in the advance/retreat grooves 10N in the second shaft portion 10B. When the holder member 12 advances/retreats in the axial direction with respect to the retaining member 10, the projections 121T of the second holder member 12 advance/retreat in the advance/retreat grooves 10M in the first shaft portion 10A.

As shown in Figs. 4 to 6, the advance/retreat grooves 111A, 121A are grooves that extend axially on the outer circumferential surfaces of the pillar-shaped parts 111, 121 at substantial centers of the pillar-shaped parts 111, 121 in the circumferential direction. These advance/retreat grooves 111A, 121A are formed from just before the proximal ends of the pillar-shaped parts 111, 121 to just before the distal ends of the pillar-shaped parts 111, 121.

Based on a viewing direction in which the bottom sides of the holder members 11, 12 are viewed from the opening sides thereof, tapered grooves 111B, 121B are formed on the left rotation sides of the advance/retreat grooves 111A, 121A. The tapered grooves 111B, 121B extend along the circumferential direction, gradually become deeper toward the left rotation sides based on the aforementioned viewing direction, and open to the side surfaces of the pillar-shaped parts 111, 121.

The tapered grooves 111C, 121C are provided along the axial direction on the distal end portions of the pillar-shaped parts 111, 121 where the advance/retreat grooves 111A, 121A are not formed. In the circumferential direction, the positions of the tapered grooves 111C, 121C coincide with the positions of the advance/retreat grooves 111A, 121A. The tapered grooves 111C, 121C gradually become deeper toward the distal end side in the axial direction, and open to the distal end surfaces of the pillar-shaped parts 111, 121.

As shown in Figs. 6 to 8, the surrounding sleeve 13, which is made of polycarbonate, has a substantially cylindrical shape and is housed in the holder members 11, 12 while surrounding the slider portions 110, 120. The surrounding sleeve 13 has a first formation portion 131 and a second formation portion 132. With a middle portion 130 serving as the axial center, the first formation portion 131 and the second formation portion 132 are respectively assembled into the first holder member 11 and the second holder member 12. In each of the first formation portion 131 and the second formation portion 132, six lock pieces 133 are provided at a substantially equal interval in the circumferential direction. Each lock piece 133 is formed by cutting the outer circumferential wall of the first formation portion 131 or the second formation portion 132 into a squared-C shape, in such a manner that the root end of each lock piece 133 is located in vicinity to the middle portion 130. In the circumferential direction, the positions at which the lock pieces 133 of the first formation portion 131 are formed substantially coincide with the positions at which the lock pieces 133 of the second formation portion 132 are formed.

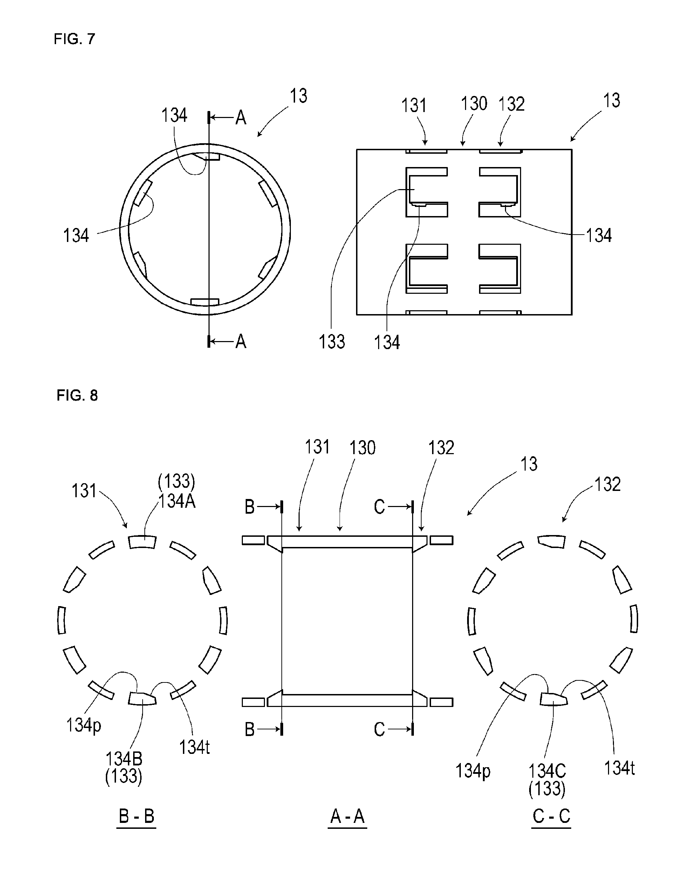

As shown in Figs. 7 and 8, every lock piece 133 has a hook-like part 134 projecting inward at the distal end side thereof (see the A-A cross-section in Fig. 8). The hook-like parts 134 have a substantially wedge-shaped cross-section. More specifically, the height of the hook-like parts 134 in the projecting direction gradually increases toward the middle portion 130.

As shown in Fig. 8, the shape and configuration of these hook-like parts 134 differ between the first formation portion 131 and the second formation portion 132. In the first formation portion 131, there are two types of hook-like parts 134. As shown in the B-B cross-section in Fig. 8, the height of the first hook-like parts 134A in the projecting direction (a direction toward the inside) is substantially constant in the circumferential direction. As shown in the B-B cross-section in Fig. 8, each second hook-like part 134B includes a part 134p and an inclined part 134t. The height of the part 134p in the projecting direction (the direction toward the inside) is substantially constant in the circumferential direction. The height of the inclined part 134t in the projecting direction gradually decreases in the circumferential direction. In each second hook-like part 134B of the first formation portion 131, the inclined part 134t is arranged at the left rotation side in the B-B cross-section in Fig. 8.

The hook-like parts 134C of the second formation portion 132 are all formed in accordance with the same specifications as shown in Fig. 8. As shown in a C-C cross-section in Fig. 8, each hook-like part 134C includes a part 134p and an inclined part 134t. The height of the part 134p in the projecting direction (the direction toward the inside) is substantially constant. The height of the inclined part 134t in the projecting direction gradually decreases in the circumferential direction. Similarly to the second hook-like parts 134B of the first formation portion 131, in each hook-like part 134C of the second formation portion 132, the inclined part 134t is arranged at the left rotation side in the C-C cross-section in Fig. 8. When assembling the holder members 11, 12 with respect to the surrounding sleeve 13, the hook-like parts 134 are inserted in the advance/retreat grooves 111A, 121A after climbing over the ends of the advance/retreat grooves 111A, 121A by using the tapered grooves 111C, 121C (see Fig. 6) provided in the holder members 11, 12.

In the pre-filled syringe 1A (manufactured state) according to the present example with the above-described component configurations, the attachment portion 125 of the second holder member 12 is threaded into the gasket 25 and fixed to the vial 2 (see Fig. 1). The first holder member 11 holds the retaining member 10 with the projections 111T circumscribing the small-diameter portion 105. The second holder member 12 holds the retaining member 10 with the projections 121T circumscribing the small-diameter portion 105. The first holder member 11 and the second holder member 12 are coaxially joined via the retaining member 10. More specifically, the first holder member 11 and the second holder member 12 are joined via the retaining member 10 in such a manner that the slider portions 110, 120 (pillar-shaped parts 111, 121) thereof do not overlap with one another in the axial direction (see Fig. 5). Furthermore, the surrounding sleeve 13 is arranged around the outer circumferences of the slider portions 110, 120 of the first and second holder members 11, 12 (see Fig. 6).

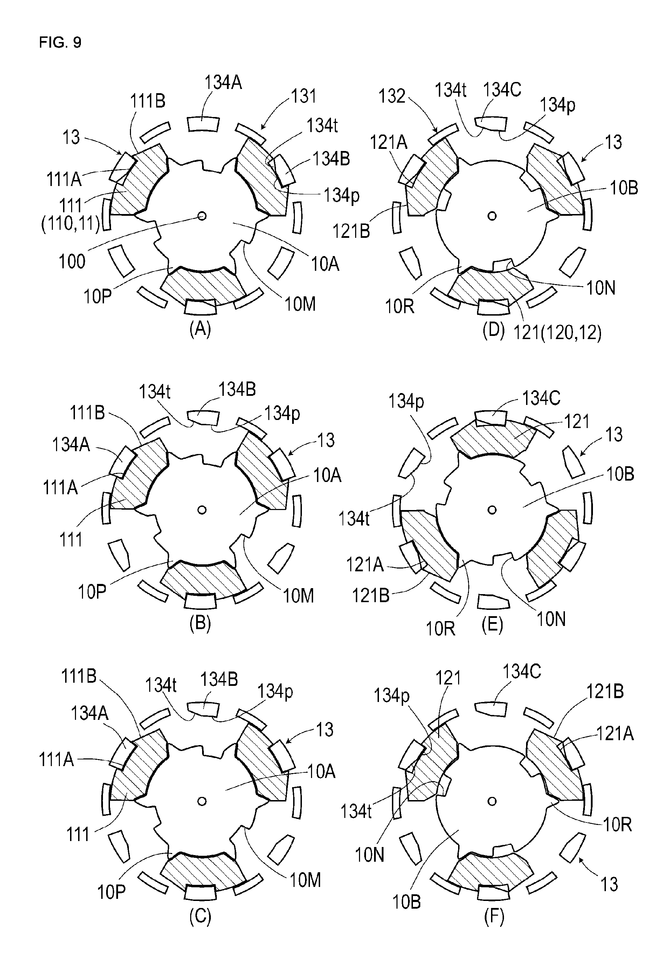

The slider portions 110, 120, the surrounding sleeve 13, and the retaining member 10 in the manufactured state are arranged as shown in Figs. 9(A) and 9(D) in the uppermost row in Fig. 9. Note that in this Fig. 9, Figs. 9(A) to 9(C) in the left column are cross-sectional views showing cross-sections including the tapered grooves 111B in the first holder member 11 as viewed from the distal end side of the pre-filled syringe 1A. In this Fig. 9, Figs. 9(D) to 9(F) in the right column are cross-sectional views showing cross-sections including the tapered grooves 121B in the second holder member 12 as viewed from the distal end side of the pre-filled syringe 1A. Figs. 9(A) to 9(C) show cross-sections of the first formation portion 131 in the surrounding sleeve 13, the slider portion 110 in the holder member 11, and the first shaft portion 10A in the retaining member 10, from the outer circumferential side. Figs. 9(D) to 9(F) show cross-sections of the second formation portion 132 in the surrounding sleeve 13, the slider portion 120 in the holder member 12, and the second shaft portion 10B in the retaining member 10, from the outer circumferential side.

As shown in Figs. 9(A) and 9 (D) in Fig. 9, in the pre-filled syringe 1A in the manufactured state, the positions of the pillar-shaped parts 111 in the first holder member 11 substantially coincide with the positions of the pillar-shaped parts 121 in the second holder member 12 in the circumferential direction (see Fig. 5). In this state, the pre-filled syringe 1A is in the axially non-retractable state as the distal end surfaces of the pillar-shaped parts 111 face the distal end surfaces of the pillar-shaped parts 121. The retaining member 10 is completely housed in the first and second holder members 11, 12, and the injection needle 100A at the distal end side and the perforation needle 100B at the side of the vial 2 are stowed as well (see Fig. 1).

As shown in Fig. 9(A) in Fig. 9, the outer circumferential surface of the first shaft portion 10A in the retaining member 10 is segmented into six surface regions in the circumferential direction by the ridge portions 10P; out of these six surface regions, surface regions where the advance/retreat grooves 10M are not formed are circumscribed by the pillar-shaped parts 111 in the first holder member 11. A rotation of the first holder member 11 relative to the retaining member 10 is restricted by the ridge portions 10P provided at six places in the circumferential direction. The second hook-like parts 134B of the surrounding sleeve 13 (first formation portion 131) are inserted in the advance/retreat grooves 111A in the pillar-shaped parts 111. As described above, the inclined parts 134t are formed on these second hook-like parts 134B at the left rotation sides in Fig. 9(A).

On the other hand, as shown in Fig. 9(D) in Fig. 9, the outer circumferential surface of the second shaft portion 10B in the retaining member 10 is segmented into three surface regions by the ridge portions 10R; these three surface regions are each circumscribed by the corresponding pillar-shaped part 121 in the second holder member 12. In Fig. 9(D), the second holder member 12 has completely rotated toward the right rotation side with respect to these three surface regions that each span across approximately 120 degrees in the circumferential direction. The hook-like parts 134C in the surrounding sleeve 13 (second formation portion 132) are inserted in the advance/retreat grooves 121A provided on the outer circumferential surfaces of the pillar-shaped parts 121. As described above, the inclined parts 134t are formed on these hook-like parts 134C at the left rotation sides in Fig. 9(D).

The use of the pre-filled syringe 1A according to the present example will now be described. In order to use the pre-filled syringe 1A, the second holder member 12 is rotated with respect to the first holder member 11 and the retaining member 10 by 60 degrees in the left rotation direction in Fig. 9. The width of each pillar-shaped part 121 in the second holder member 12 in the circumferential direction is equivalent to approximately 60 degrees in the circumferential direction. On the other hand, as shown in Fig. 9(D) in Fig. 9, in the second shaft portion 10B of the retaining member 10 circumscribed by the pillar-shaped parts 121, the ridge portions 10R are provided in three places at a substantially equal interval in the circumferential direction, so as to segment the outer circumferential surface into surface regions that each span across approximately 120 degrees in the circumferential direction. In the state where the pillar-shaped parts 121 circumscribe the second shaft portion 10B in the retaining member 10, the second holder member 12 is rotatable relative to the retaining member 10 within a range of approximately 60 degrees. A leftward rotation of the second holder member 12 causes the surrounding sleeve 13 to rotate leftward due to the engagement between the advance/retreat grooves 121A and the hook-like parts 134C.

As shown in Fig. 9(A), on the second hook-like parts 134B that engage with the advance/retreat grooves 111A in the first slider portion 110, the inclined parts 134t are formed at the left rotation sides. Furthermore, the tapered grooves 111B extending along the circumferential direction are formed in the pillar-shaped parts 111 at the right rotation sides. When the surrounding sleeve 13 is caused to rotate leftward by the leftward rotation of the second holder member 12 as described above, the second hook-like parts 134B exit the advance/retreat grooves 111A by using the inclined parts 134t, and the first hook-like parts 134A climb into the advance/retreat grooves 111A by using the inclined bottom surfaces of the tapered grooves 111B, as shown in Figs. 9(A) and 9(B).

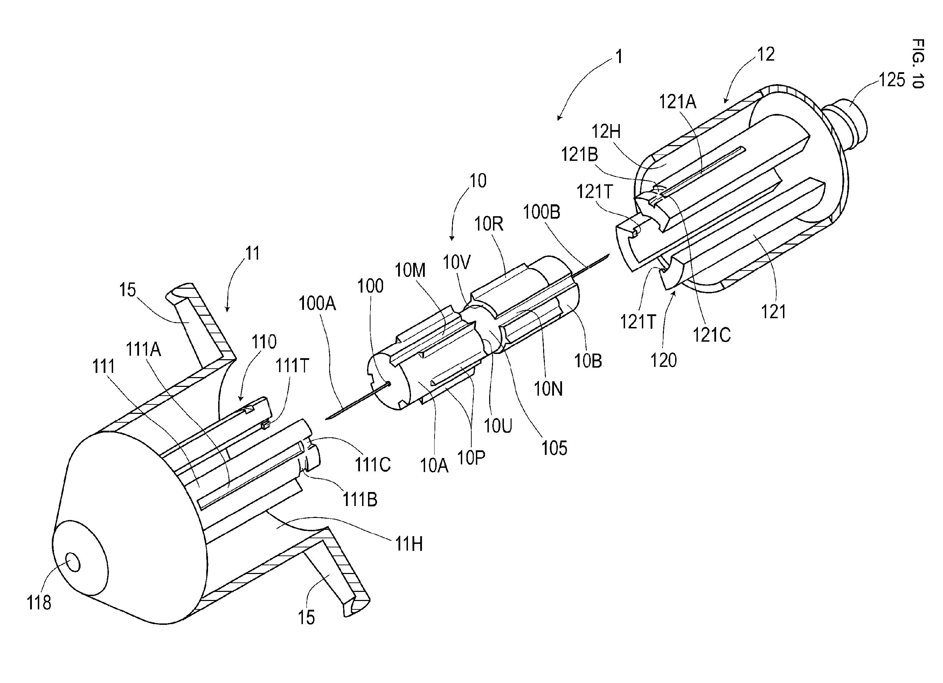

When the second holder member 12 is rotated leftward together with the surrounding sleeve 13 in the above-described manner, an injection state of Figs. 9(B) and 9(E) can be realized. In this injection state, the pillar-shaped parts 111 of the first holder member 11 and the pillar-shaped parts 121 of the second holder member 12 are positioned alternately in the circumferential direction. In the state where the pillar-shaped parts 111, 121 are thus arranged alternately, the needle unit 1 is retractable in the axial direction (see Figs. 10 to 12).

For example, first, the needle unit 1 is retracted in the axial direction with a pointing finger and a middle finger placed around the finger grips 15 and a thumb placed on a bottom surface 210 of the vial 2 (see Fig. 2). Once the needle unit 1 has been retracted in the axial direction to the extent that the length thereof in the axial direction is shorter than the total length of the retaining member 10 inclusive of the injection needle 100A and the perforation needle 100B, the perforation needle 100B projects from the attachment portion 125 of the second holder member 12, and the injection needle 100A projects from the distal end of the first holder member 11. The perforation needle 100B penetrates through the wall 251 in the gasket 25 (see Fig. 3), and the distal end thereof reaches the inside of the vial 2. When the needle unit 1 is retracted in the axial direction to the fullest extent, the injection needle 100A and the perforation needle 100B project to the fullest extent. By pushing the needle unit 1 toward the bottom side of the vial 2, the pre-filled syringe 1A is further retracted in the axial direction, and the gasket 25 moves in the forward direction. This enables injection of the drug solution.

As shown in Fig. 9(B), in the pre-filled syringe 1A in the injection state, the first hook-like parts 134A of the surrounding sleeve 13 (first formation portion 131) are inserted in the advance/retreat grooves 111A in the first slider portion 110. Furthermore, as shown in Fig. 9(E) in Fig. 9, the second holder member 12 has completely rotated toward the left rotation side with respect to the outer circumferential surface that is segmented by the ridge portions 10R into surface regions that each span across 120 degrees in the circumferential direction. As the leftward rotation of the second holder member 12 causes the surrounding sleeve 13 to rotate from the state of Fig. 9(D) to the state of Fig. 9(E) in Fig. 9, the hook-like parts 134C inserted in the advance/retreat grooves 121A in the second slider portion 120 are not switched. During the post-use handling, the inclined parts 134t that are provided in these hook-like parts 134C in the left rotation sides achieve extremely important operational effects.

The following describes processes performed after the use of the pre-filled syringe 1A according to the present example. After the injection is completed, the pre-filled syringe 1A is extended in the axial direction by withdrawing the vial 2 from the needle unit 1. The gasket 25 has a large resistance to sliding when it retreats in the axial direction inside the vial 2. Therefore, the aforementioned withdrawal first causes the needle unit 1 to extend in the axial direction. The needle unit 1 is extended until the hook-like parts 134A reach the ends of the advance/retreat grooves 111A in the first slider portion 110, and until the hook-like parts 134C reach the ends of the advance/retreat grooves 121A in the second slider portion 120.

Furthermore, extending the pre-filled syringe 1A in the axial direction allows the gasket 25 to retreat inside the vial 2. The gasket 25 retreats until it reaches the fall prevention portion 23.

As described above, the pre-filled syringe 1A is extended in the axial direction until the hook-like parts 134A, 134C reach the ends of the advance/retreat grooves 111A, 121A in the slider portions 110, 120, and when the gasket 25 reaches the fall prevention portion 23. Further extension is impossible unless physical breakage occurs (first extension restriction mechanism). The extension of the needle unit 1 in the axial direction is also restricted by the engagement between the projections 111T, 121T provided in the pillar-shaped parts 111, 121 and the end surfaces 10U, 10V of the retaining member 10 (second extension restriction mechanism). This makes it possible to prevent separation of the holder members 11, 12 with high reliability.

In the state where the hook-like parts 134A, 134C have respectively reached the ends of the advance/retreat grooves 111A, 121A after withdrawing the vial 2 from the needle unit 1 in the above-described manner, the second holder member 12 is rotated again by 60 degrees in the right rotation direction in Fig. 9. At this time, as described above, hook-like parts that are inserted in the advance/retreat grooves 111A in the first slider portion 110 are the first hook-like parts 134A with no inclined parts 134t (Fig. 9(B) in Fig. 9). These first hook-like parts 134A cannot exit the advance/retreat grooves 111A even with the occurrence of a rotational force relative to the first holder member 11. Therefore, in the state of Fig. 9(B) in Fig. 9, a relative rotation of the first holder member 11 and the surrounding sleeve 13 is restricted.

On the other hand, as shown in Fig. 9(E), in the hook-like parts 134C inserted in the advance/retreat grooves 121A in the second slider portion 120, the inclined parts 134t are formed at the left rotation sides. Therefore, a rightward rotation of the second holder member 12 allows the hook-like parts 134C to exit the advance/retreat grooves 121A by using the inclined parts 134t. When the second holder member 12 is rotated rightward relative to the surrounding sleeve 13, the second slider portion 120 approaches new hook-like parts 134C. As all the hook-like parts 134C include the inclined parts 134t at the left rotation sides, they can climb into the advance/retreat grooves 121A in the slider portion 120 by using these inclined parts 134t. Therefore, when the second holder member 12 is rotated rightward in the state of Fig. 9(E), only the second holder member 12 can be rotated rightward without causing the surrounding sleeve 13 to rotate.

The aforementioned rightward rotation of the second holder member 12 in the injection state of Figs. 9(B) and 9(E) leads to a disposal state shown in Figs. 9(C) and 9(F) in Fig. 9. In this disposal state, the positions of the pillar-shaped parts 111 in the first holder member 11 substantially coincide with the positions of the pillar-shaped parts 121 in the second holder member 12 in the circumferential direction. That is to say, in this state, the pre-filled syringe 1A is in the axially non-retractable state as the distal end surfaces of the pillar-shaped parts 111 face the distal end surfaces of the pillar-shaped parts 121.

As shown in Fig. 9(C), the first hook-like parts 134A with no inclined parts 134t are inserted in the advance/retreat grooves 111A in the first slider portion 110. Therefore, in this state, the surrounding sleeve 13 cannot be rotated relative to the first holder member 11. Furthermore, as six ridge portions 10P are formed on the outer circumference of the first shaft portion 10A, the first holder member 11 cannot be rotated relative to the retaining member 10, either.

In addition, as shown in Fig. 9(F), the hook-like parts 134C, which have the inclined parts 134t on the left rotation sides, are inserted in the advance/retreat grooves 121A in the second slider portion 120. In Fig. 9(F), a leftward rotation of the second holder member 12 is restricted due to the engagement between the advance/retreat grooves 121A and the hook-like parts 134C, and a rightward rotation of the second holder member 12 is restricted by the ridge portions 10R with which the right rotation sides of the pillar-shaped parts 121 are in contact. Therefore, the second holder member 12 cannot be rotated relative to the retaining member 10.

As described above, upon shifting to the states of Figs. 9(C) and 9(F) by rotating the second holder member 12 rightward after use, the action of a rotation restriction mechanism made up of the surrounding sleeve 13 and the like does not allow a relative rotation of the first holder member 11 and the second holder member 12.

Furthermore, in the state of Fig. 9(C), the positions of the advance/retreat grooves 10M in the first shaft portion 10A and the positions of the projections 111T in the first holder member 11 differ in the circumferential direction. In this way, the projections 111T engage with the end surface 10U between the small-diameter portion 105 and the first shaft portion 10A, thereby restricting withdrawal of the first holder member 11 from the retaining member 10 in the axial direction. Similarly, in the state of Fig. 9(F), the positions of the advance/retreat grooves 10N in the second shaft portion 10B differ from the positions of the projections 121T in the second holder member 12 in the circumferential direction. In this way, the projections 121T engage with the end surface 10V between the small-diameter portion 105 and the second shaft portion 10B, thereby restricting withdrawal of the second holder member 12 from the retaining member 10 in the axial direction.

The pre-filled syringe 1A according to the present example configured in the above manner is extremely compact and very safe. This pre-filled syringe 1A is an excellent product that reduces the time and efforts required in administering the injection to a great extent and that can be placed in a very safe disposal state via a simple operation after the injection is completed.

It should be noted that the needle unit 1 is not limited to being made of polycarbonate as in the present example, and may be made of various types of materials such as polypropylene, polyethylene, polyurethane, and polyethylene terephthalate. Furthermore, the retaining member 10 or the holder members 11, 12 may be made of biodegradable plastic. In this case, disposal of the pre-filled syringe 1A is further facilitated.

### (Reference Examples)

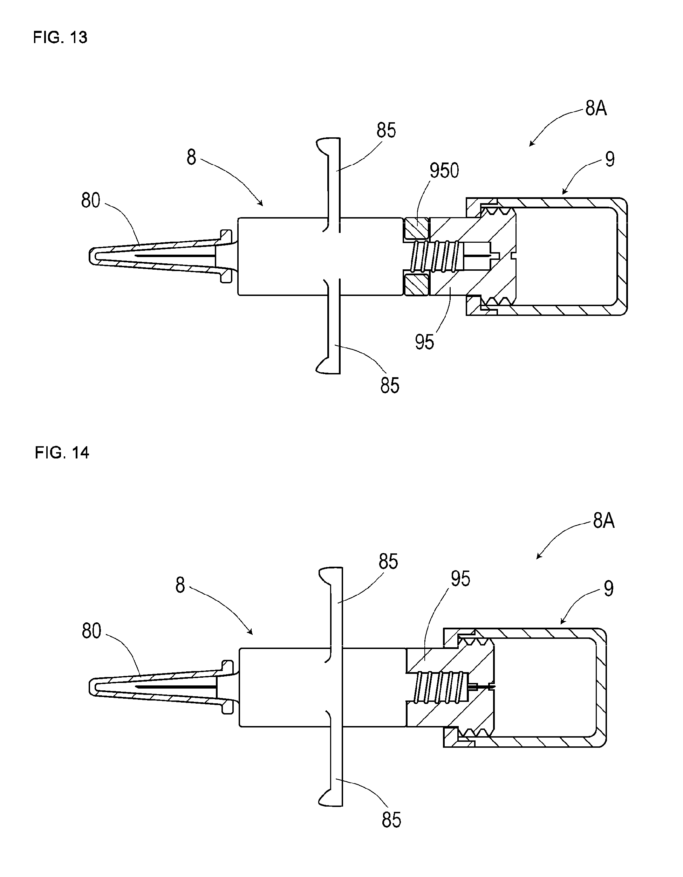

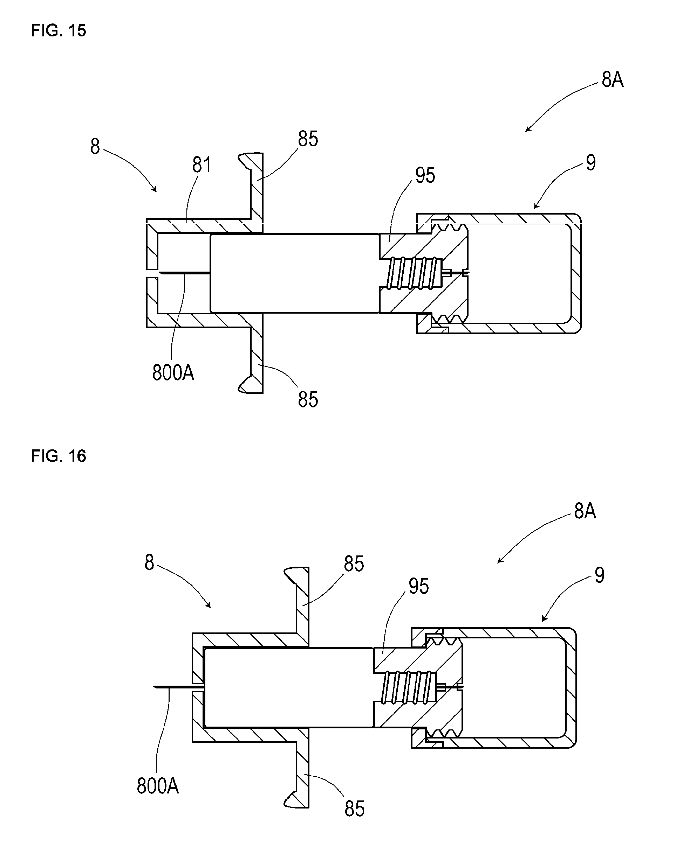

The present reference examples provide another pre-filled syringe 8A that uses a vial 9 similarly to the working example. Figs. 13 and 14 show a first reference example, and Figs. 15 and 16 show a second reference example.

Fig. 13 shows a pre-filled syringe 8A in a manufactured state according to the first reference example. In this pre-filled syringe 8A, a needle unit 8 is threaded into a gasket 95 in an incomplete manner due to a substantially U-shaped spacer member 950 provided between the needle unit 8 and the gasket 95. Unlike the needle unit 1 according to the working example, the needle unit 8 according to the present reference example has a fixed length in the axial direction.

At the time of injection, as shown in Fig. 14, the spacer member 950 is removed, and then the needle unit 8 is threaded completely into the gasket 95. Thereafter, the injection can be administered by removing a needle cap 80 that is attached to and cover the distal end.

Fig. 15 according to the second reference example shows the state where a needle unit 8 has been threaded into a gasket 95 after removing a spacer member (not shown in the drawings) similar to the one according to the first reference example. In this state, an injection needle 800A is stowed in a needle cover 81 biased towards the distal end side. By retracting a pre-filled syringe 8A in the axial direction with fingers placed around a pair of finger grips 85 provided to the needle cover 81 and on the bottom surface of a vial 9, an injection needle 800A projects from the distal end side as shown in Fig. 16. By further retracting the pre-filled syringe 8A in the axial direction, a drug solution can be injected from the projecting injection needle 800A.

Although specific examples of the present invention, including the working example, have been described above in detail, these specific examples merely disclose examples of techniques embraced in the claims. It goes without saying that comprehension of the claims should not be limited by configurations and numeric values according to the specific examples. The claims encompass techniques that are conceived by modifying or changing the specific examples in a wide variety of ways using known techniques and knowledge of a person skilled in the art.

### Description of Symbols

- 1: needle unit (needle housing unit)
- 1A: pre-filled syringe
- 10: retaining member
- 11, 12h: older member
- 11H, 12H: hollow portion
- 100A: injection needle
- 100B: perforation needle
- 110, 120: slider portion
- 111, 121: pillar-shaped part
- 125: attachment portion
- 13: surrounding sleeve
- 134: hook-like part
- 15: finger grip
- 2: vial (drug solution container)
- 210: bottom surface
- 230: opening
- 25: gasket (closing member)
- 250: attachment hole
- 251: wall

## Claims

1. A pre-filled syringe, comprising:
a drug solution container that is filled with a drug solution and has a shape of a cylinder with a bottom;
a closing member that closes an opening of the drug solution container in a manner movable in an axial direction, which is equivalent to a boring direction of the cylindrical shaped drug solution container; and
a needle housing unit that is attached to the closing member so as to allow the closing member to be pushed into the drug solution container, wherein
the needle housing unit includes:
a substantially columnar retaining member with an injection needle projecting from one end thereof in the axial direction and a perforation needle projecting from the other end thereof in the axial direction;
a first holder member provided with a first hollow portion having a bottom and with a first slider portion, the first slider portion being able to hold the retaining member along the axial direction, which is equivalent to a direction from a bottom side toward an opening side of the first hollow portion, using at least two pillar-shaped parts, the at least two pillar-shaped parts extending from the bottom side of the first hollow portion along the axial direction and circumscribing an outer circumferential surface of the retaining member; and
a second holder member provided with a second hollow portion having a bottom, with a second slider portion, and with an attachment portion, the second slider portion being able to hold the retaining member using at least two pillar-shaped parts that extend from a bottom side of the second hollow portion along the axial direction, which is equivalent to a direction from a bottom side toward an opening side of the second hollow portion, and that circumscribe the outer circumferential surface of the retaining member, the attachment portion being provided on a bottom end of the second holder member for the closing member,
the first holder member and the second holder member are rotatable relative to each other around the retaining member in a state where each pillar-shaped part in the first slider portion or the second slider portion holding the retaining member does not overlap with any of the pillar-shaped parts in the other slider portion in the axial direction,
in a state where the pillar-shaped parts in the slider portions are arranged alternately around the retaining member, the first holder member and the second holder member are retractable in the axial direction through insertion of one of the holder members into the hollow portion in the other holder member, whereas in a state where distal end surfaces of the pillar-shaped parts in one of the slider portions face distal end surfaces of the pillar-shaped parts in the other slider portion, the first holder member and the second holder member are not retractable in the axial direction, and
when the first holder member and the second holder member holding the retaining member are retracted in the axial direction, the perforation needle penetrates through the closing member and projects inside the drug solution container, and the injection needle projects toward the outside.

2. The pre-filled syringe according to claim 1, further comprising
a surrounding sleeve having a substantially cylindrical shape, the surrounding sleeve restricting a relative rotation of the first holder member and the second holder member around the retaining member in a state where the surrounding sleeve surrounds the first slider portion and the second slider portion.

3. The pre-filled syringe according to claim 2, wherein
the needle housing unit is in an axially non-retractable state in a manufactured state, and is placed in an axially retractable state by rotating the first holder member and the second holder member relative to each other around the retaining member during use, the axially non-retractable state being a state where the needle housing unit is not retractable in the axial direction, and the axially retractable state being a state where the needle housing unit is retractable in the axial direction,
after use, the needle housing unit can be placed in the axially non-retractable state again by rotating the first holder member and the second holder member relative to each other after extending the first holder member and the second holder member in the axial direction to positions where each pillar-shaped part in the first slider portion or the second slider portion does not overlap with any of the pillar-shaped parts in the other slider portion in the axial direction, and
the surrounding sleeve allows the relative rotation of the first holder member and the second holder member around the retaining member in the manufactured state, and restricts the relative rotation of the first holder member and the second holder member once the needle housing unit has shifted from the axially retractable state to the axially non-retractable state again after use.

4. The pre-filled syringe according to claim 2 or 3, wherein
the surrounding sleeve restricts a maximum extended length of the first holder member and the second holder member in the axial direction.

5. The pre-filled syringe according to any one of claims 1 to 4, wherein
the first holder member allows the second holder member to be inserted into the first hollow portion, and has finger grips on an outer circumferential side thereof, the finger grips allowing a user to place fingers therearound when administering an injection, and
the injection can be administered with fingers placed on a bottom surface of the drug solution container and around the finger grips.
